# EUROPEAN PATENT SPECIFICATION

(11) **EP 3 184 150 B1**
(45) Date of publication and mention of the grant of the patent: **19.06.2019**
(21) Application number: 16199408.2
(22) Date of filing: 22.11.2006
(51) Int. Cl.: A61K 31/5375, A61K 9/00, A61P 1/02

(54) **TREATMENT OF SUB-GINGIVAL POCKET INFECTIONS**
BEHANDLUNG VON ZAHNFLEISCHTASCHENINFEKTIONEN
TRAITEMENT DES INFECTIONS DE POCHE SOUS-GINGIVALE

(30) Priority: 22.11.2005 GB 0523745; 09.01.2006 GB 0600324
(43) Date of publication of application: 28.06.2017
(62) Divisional of application: 06808626.3
(73) Proprietor: Maelor Laboratories Limited, Chippenham Wiltshire SN15 2BB (GB); Merial, Inc., Duluth, GA 30095 (US)
(72) Inventor: PERSSON, Gosta, Rutger, CH-3010 Berne (CH)
(74) Representative: D Young & Co LLP

(56) References cited:
- WO-A-92/07548
- WO-A-92/08442
- SCANDINAVIAN JOURNAL OF DENTAL RESEARCH, COPENHAGEN, DK, vol. 102, no. 1, 1994, pages 17-25, XP008067770, ISSN: 0029-845X
- ORAL MICROBIOLOGY AND IMMUNOLOGY, MUNKSGAARD, COPENHAGEN, DK, vol. 8, no. 1, 1993, pages 36-41, XP008067768, ISSN: 0902-0055
- D ETIENNE: "Locally delivered antimicrobials for the treatment of chronic periodontitis", ORAL DISEASES, vol. 9, no. s1, 1 June 2003 (2003-06-01), pages 45-50, XP055219986, GB ISSN: 1354-523X, DOI: 10.1034/j.1601-0825.9.s1.8.x

## Description

### Field of the Invention

The present invention relates to a morpholino compound for use in the treatment of sub-gingival infection.

### Background to the Invention

Gum disease is a major cause of ill-health, worldwide, and commonly leads to tooth decay. The two major forms of gum disease, gingivitis and periodontitis, result from bacterial plaque formation in the oral cavity, in particular on the teeth along the margin of the gums.

A sub-ginglval Infection begins when bacteria at the tooth-gingiva boundary cause the gingival tissue (i.e. the gums) to separate from the tooth, forming a sub-gingival pocket. Bacteria collect in this pocket, causing gingival swelling that traps bacteria in the sub-glngival pocket. Further inflammation exacerbates the infection and eventually both gingival tissue and bone are destroyed. Treating sub-gingival infections is particularly problematic as the infecting bacteria are hard to access and it is difficult to apply anti-bacterial agents to the site of infection. Furthermore, there is thought to be a flow of gingival fluids out of the sub-gingival pocket, which hinders entry of any therapeutic agent into the site of infection.

Sub-gingival infections are characteristic of a number of oral infections, including adult and juvenile chronic periodontitis, early-onset periodontitis, necrotizing ulcerative periodontal diseases and acute pericoronitis.

The anti-bacterial agent chlorhexidine is used to treat oral Infections. When used to treat sub-gingival pocket infections, chlorhexidine suffers unsightly staining and a loss of taste and further suffers from low patient compliance due to an unpleasant stinging sensation that occurs on its application. This can be extremely painful.

Infections in sub-gingival pockets are often characterized by the presence of a combination of bacterial species known as "The Red Complex". This comprises *Tannerella forsythensis, Porphyromonas gingivalis* and *Treponema denticola* and it is thought that the components of the red complex are synergistic (see for example, Holt and Ebersole, Periodontol 2000. 2005;38:72-122). *Actinomyces* species such as *Actinobacillus actinomycetemcomitans* are also implicated in oral (sub-gingival) Infections.

Changes in the bacterial flora, and the interactions between different bacterial species, are thought to influence the progression of oral disease (see for example Socransky and Haffajee, Periodontol 2000. 2005;38:135-87).

*Actinobacillus actinomycetemcomitans* is a non-motile, gram-negative, capnophilic, fermentative coccobacillus that has been implicated in the aetiology and pathogenesis of juvenile and adult periodontitis as well as systemic infections. *Actinobacillus actinomycetemcomitans* has serotypes a-e. Strain Y4 is serotype b. It has been shown that antibodies reactive to *Actinobacillus actinomycetemcomitans* serotype b (i.e. strain Y4) lipopolysaccharide correlate with reduction of attachment loss in generalised early onset peridontitis patients (Califano J.V. et al, Infection and Immunity, Sept. 1996, p.3908-391 0). Most of the antibody reactive with *Actinobacillus actinomycetemcomitans* serotype b LPS is IgG2 (Lu et al, Infect Immun 1993 Jun; 61(6): 2400-7), and serum IgG2 is decreased in smokers (Quinn, S. et al, Infect. Immun. 64:2500-2505). It should be noted that *Actinobacillus actinomycetemcomitans* is sometimes referred to in the art as *Aggregatibacter actinomycetemcomitans.*

Besides periodontal infections, *Actinobacillus actinomycetemcomitans* is occasionally isolated from severe systemic infections (Van Winkelhoff A.J. and Slots J. Periodontol. 2000 1999;20:122-135).

*Treponema denticola* infection has been proven to be closely associated with periodontal diseases such as early onset periodontitis, necrotizing ulcerative gingivitis and acute pericoronitis (Chan EC, McLaughlin R. Oral Microbiol Immunol 2000;15:1-9; Takeuchi Y, Umeda M, Sakamoto M, et al. J Periodontol 2001;72:1354-1363).

Particular attention has been paid recently to the clinical significance of coinfection of periodontal bacterial pathogens. *P*. *gingivalis, A. actinomycetemcomitans* and *T. denticola* as well as other sub-gingival bacteria form a mixed infection, which causes more serious periodontal destruction than a single infection (Langendijk-Genevaux PS et al. J Clin Periodontol 2001;28:1151-1157; Shiloah J, et al. J Periodontol 2000;71:562-567).

The morpholino compound commonly referred to as Delmopinol is known to be useful in treating and preventing dental plaque and mild gingivitis, as part of a normal oral health routine (see, for example, Collaert et al, Scand J Dent Res 1994; 102: 17-25). The microbiology of early supragingival plaque development after delmopinol treatment is disclosed by Collaert et al, Oral Microbiol Immunol 1993:8:36-41. Improved anti-plaque compositions comprising a combination of a morpholino amino alcohol and a chelating agent are disclosed by WO-A-92/07548. Improved anti-plaque compositions comprising a combination of a morpholino amino alcohol and an anti-microbial agent are disclosed by WO-A-92/08442.

Current treatments for sub-gingival infection, for example in chronic periodontitis, commonly involve surgical intervention, to remove the infected tissue and bacterial plaque from the sub-gingival pocket and to reduce the depth of the pocket. Even if open surgery is not involved, sub-gingival scaling and root planing is a standard treatment; locally delivered antiseptics such as anti-bacterial-soaked pads may also be inserted into the sub-gingival pocket (see for example Etienne, Oral Dis. 2003;9 Suppl 1:45-50) These treatments are expensive, time-consuming, inconvenient and painful. To date, simple anti-bacterial treatments have not been successful in treating sub-gingival infections. There is a clear need for an improved method of clearing an infection from a sub-gingival pocket.

### Summary of the Invention

The present invention is based on the surprising discovery that a compound of formula (I) is efficacious in treating infection of a sub-gingival pocket. Sub-gingival infections commonly present in a number of periodontal diseases, and a compound of formula (I) can be used to treat these diseases. This finding is surprising, as compounds of formula (I) are known to have only very weak anti-microbiai properties and would therefore not be expected to be effective in treating a well-established infection.

According to a first aspect of the invention, a morpholino compound having the general formula (I) 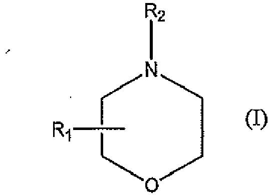 wherein R1 is a straight or branched alkyl group containing 8 to 16 carbon atoms at the 2- or 3- position of the morpholino ring, and R2 is a straight or branched alkyl group containing 2 to 10 carbon atoms, substituted with a hydroxy group except in the alpha-position, or a pharmaceutically acceptable salt thereof is used in the manufacture of a medicament for the treatment of an infected sub-gingival pocket.

Also disclosed is a kit for treating an infection in a sub-gingival pocket comprises a compound having the general formula (I) and instructions that the compound is to be used for treatment of the infection.

### Description of the Drawings

The invention is described with reference to the following drawings, wherein:
Figure 1 illustrates the sub-gingival reduction in bacterial load , in four selected sub-gingival pockets, after a 14 day course of delmopinol rinsing (once daily); and
Figure 2 illustrates the reduction of *Treponema denticola* and *Aggregatibacter actinomycetemcomitans* after the 14 day course of delmopinol treatment.

### Detailed Description of the Invention

A morpholino compound of formula (I) can be used to treat sub-gingival pockets infected with bacteria. A morpholino compound according to the invention has the general formula (I) 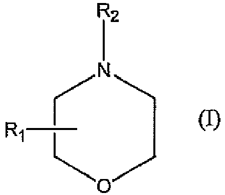 wherein R1 is a straight or branched alkyl group containing 8 to 16 carbon atoms at the 2- or 3- position of the morpholino ring, and R2 is a straight or branched alkyl group containing 2 to 10 carbon atoms, substituted with a hydroxy group except in the alpha-position, or pharmaceutically acceptable salts thereof. In a preferred embodiment, the sum of the carbon atoms in the groups R1 and R 2 of the morpholino compound is at least 10, preferably between 10 and 20. In a further preferred embodiment, the R 2 group terminates with the hydroxy group.

The claimed morpholino compounds are known *per se* and can be manufactured by any known method, for example that disclosed in US5,082,653 and WO90/14342.

The preferred morpholino compound for use in the invention is 3-(4-propyl-heptyl)-4-(2-hydroxyethyl)morpholine, which is commonly known as Delmopinol (CAS No. 79874-76-3).

The morpholino compounds can be used in their free base form or as a pharmaceutically acceptable salt thereof. Examples of pharmaceutically acceptable salts are the salts of acids such as acetic acid, phosphoric acid, boric acid, hydrochloric acid, maleic acid, benzoic acid, citric acid, malic acid, oxalic acid, tartaric acid, succinic acid, glutaric acid, gentisic acid, valeric acid, gallic acid, beta-resorcyclic acid, acetyl salicylic acid, salicylic acid, perchloric acid, barbituric acid, sulfanilic acid, phytic acid, p-nitro benzoic acid, stearic acid, palmitic acid, oleic acid, myristic acid, lauric acid and the like. The most preferred salts are those of hydrochloric acid. A preferred compound is delmopinol hydrochloride (CAS No. 98092-92-3).

As used herein, the term "infection" is to be given its usual meaning in the art, i.e. the detrimental colonization of a cell, tissue, organ or body by a foreign species. According to the current invention, the foreign species are bacteria.

The site of the infection is a "sub-gingival pocket". The term "sub-gingival pocket" is known in the art to refer to a pit or hollow that is formed between a tooth and the gingival tissue associated with that tooth. Sub-gingival pockets are usually a symptom of advanced, chronic or severe forms of gingivitis. The pocket is usually formed due to the gingival tissue separating from the tooth in response to an initially mild bacterial infection at the tooth-gingival boundary. A small, for example less than 1mm deep pocket (measured from the apex of the pocket to the edge of the gingival tissue), may not require anti-microbial treatment as a normal oral health routine may suffice, e.g. brushing may remove the bacteria. However, sub-gingival pockets can grow up to 12mm deep or more. The more severe pockets, for example deeper than 3 mm deep or more, for example 4, 5, 6, 7 or 8 mm deep or more are likely to require anti-microbial treatment. Although a bacterial infection in a sub-gingival pocket of any size can be treated by contact with a compound of formula (I), it is preferred that deeper pockets, for example deeper than 3 mm, preferably at least 4, 5, 6, 7 or 8 mm deep or more are treated according to the current invention. These deep pockets are likely to be caused by chronic periodontal infections. Using conventional methods, an infection in these deeper pockets is likely to be difficult to treat without surgical intervention.

A further advantage of using a compound of formula (I) to treat a sub-gingival infection is that, surprisingly, administration is substantially pain free.

According to the current invention, a compound of formula (I) is used to treat an existing sub-gingival infection, wherein the sub-gingival pocket is 4 mm deep or more. This is separate from the previously known use of delmopinol as a prophylactic agent to prevent an infection arising due to the ability to prevent supra-gingival plaque formation and mild gingivitis. The compound is administered to a patient that presents with infected sub-gingival pockets. Contacting the oral cavity, and therefore the sub-gingival pockets, with a compound of formula (I) reduces the number of bacteria in the infected pocket and allows the gingival tissue to recover. In a preferred embodiment, no further treatment is required, i.e. the gingival tissue grows back to an acceptable level thereby "closing" the pocket. Application of the compound of formula (I) to the site of infection does not require the intervention of a qualified medical or dental practitioner, such as a dental hygienist or periodontist.

The current invention is applicable equally in the fields of human and animal medicine, i.e. veterinary applications are within the scope of the invention. In the veterinary embodiment, treatment of pets including cats and dogs, and treatment of farm animals including cattle and swine, are preferred embodiments.

A compound of formula (I) is useful in treating sub-gingival infections and, surprisingly, it can access the sub-gingival site of infection. It has been found that a compound of formula (I) results in a significant reduction of *T. denticola* levels (see example 1). Compounds of formula (I) are therefore surprisingly effective in reducing levels of bacteria that are components of the "Red Complex", which is a characteristic of sub-gingival microflora colonisation.

It has been also been found that a compound of formula (I) almost eliminates *A. actinomycetemcomitans* (see example 1). This bacterium is implicated in the development of the red complex and is often found in infected sub-gingival pockets.

A compound of formula (I) can be used to alter the level of specific bacteria, preferably *T*. *denticola* and/or *A*. *actinomycetemcomitans* (*A. actinomycetemcomitans* is also sometimes referred to in the art as *Aggregatibacter actinomycetemcomitans*) in the oral cavity, preferably at an infected sub-gingival site.

Without wishing to be bound by theory, it is thought that the Red complex is particularly problematic because the bacterial species within the complex interact and create a stubborn infection (see, for example, Langendijk-Genevaux *et al*, supra). Therefore reducing the level of at least one member of the Red complex will reduce the pathogenicity of this complex of bacteria and therefore effectively treat a sub-gingival infection.

Sub-gingival pocket infections commonly occur in periodontal diseases such as adult and juvenile chronic periodontitis, early-onset periodontitis, necrotizing ulcerative periodontal diseases and acute pericoronitis. It is possible to treat other symptoms of these diseases, such as infection of the (supra-gingival) tooth surface and tongue, using conventional techniques. Even if such conventional treatments are successful, the sub-gingival pocket infection is likely to remain but on a lower level. Therefore, a compound of formula (I) can be used to treat these diseases when the problematic symptom is a sub-gingival infection. Preferably, chronic periodontitis is treated by contacting the infected sub-gingival area with a compound of formula (I).

Use of a compound of formula (I) to treat a sub-gingival infection will also have a cosmetic benefit, i.e. infection-free gingival tissue is considered to be aesthetically pleasing.

The compound of formula (I) may be brought into contact with the oral cavity in a conventional way, in any suitable form or amount that achieves the desired effect, i.e. reduction of infection of a sub-gingival pocket. Preferably, the compound of formula (I) is in the form of a mouthwash, toothpaste, gel, dentifrice, gum or other similar preparation that will be apparent to the skilled person. Most preferably, the compound is in the form of an aqueous mouthwash. This can be applied to the oral cavity by the patient, without the need for medical supervision. The compound of formula (I) has the surprising ability to enter the sub-gingival pocket and treat the infection. Preferably, the mouthwash is held in the mouth for at least 5 seconds, preferably greater than 10 seconds, for example one minute or more, to allow the compound of formula (I) to access the site of infection.

The compound of formula (I) can be added to an existing toothpaste, gum, gel or mouthwash formulation. The compound of formula (I) may be added in combination with at least one anti-microbial, preferably anti-bacterial, agent. Suitable agents include the antibiotics tetracycline, doxycylcine and ampicillin. Other agents suitable for treating oral infections will be apparent to one skilled in the art. Alternatively, the compound of formula (I) is the only agent that treats the sub-gingival pocket infection in the preparation with which the oral cavity is contacted.

The compound of formula (I) may be added in combination with at least one anti-inflammatory agent. Anti-inflammatory agents are well known in the art and any may be used. Preferably, the anti-inflammatory agent is a non-steroidal anti-inflammatory drug (NSAID), such as aspirin (acetylsalicylic acid) or ibuprofen. In an alternative embodiment, a steroidal anti-inflammatory agent, for example cortisone, may be used. The compound of formula (I) may be added in combination with at least one anti-inflammatory agent and at least one other anti-microbial agent.

For the avoidance of doubt, agents in addition to a compound of formula (I) can be added to a composition for treating sub-gingival infection, for example the agents disclosed above can be included. However, additional agents are not required and, in a preferred embodiment, no additional agents that have an antimicrobial, therapeutic or other pharmacological effect, or which enhance the anti-infection effect of the compound of formula (I), such as chelating agents, are included in the composition. A preferred composition consists of, or consists essentially of, a compound of formula (I) and non-active ingredients such as solvents (e.g. water and/or alcohols), colourings, flavourings and sweeteners.

In a preferred embodiment, the level of alcohol in the composition comprising formula (I) is low, preferably less that 25% w/v, more preferably less than 20% w/v, yet more preferably less than 10% w/v, for example less than 5% w/v, 3% w/v or less than 1% w/v. In a further preferred embodiment, there is no alcohol present in the composition comprising formula (I). As used herein, the term "alcohol" refers to any alcohol that can be used in oral preparation, preferably ethanol.

The compound of formula (I) can be used at a suitable concentration, which will be apparent to the skilled person. In one embodiment, suitable concentrations of the compound of formula (I) are between 0.01% (w/v) to 10% (w/v), preferably from 0.1% (w/v) to 5% (w/v), more preferably from 1% (w/v) to 3% (w/v), for example 2% (w/v). The most preferred concentration of a compound of formula (I) is 0.2% w/v.

In one embodiment of the invention, a composition comprises, per 100kg, 98.267kg deionised water; 0.2kg delmopinol HCl; 0.01kg sodium saccharide; 0.02kg herb flavour COD 76634-34; 1.5kg 99.5% ethanol; and 0.003kg sodium hydroxide.

Mechanical agitation, preferably brushing or rubbing the area containing the infected sub-gingival pocket, can be performed simultaneously with or shortly, preferably immediately, after contacting the oral cavity with a compound of formula (I).

A kit or pack comprising a compound of formula (I) and instructions for treatment of a sub-gingival infection, together with instructions directing the user to use the compound to treat the infection, is included in the invention.

The invention is further described with reference to the following non-limiting example.

### Example 1

In this example delmopinol was used as a rinse to treat a patient who had been cared for over a 2-year period. She had been diagnosed with Morbus Crohn (Crohn's Disease) and with oral complications presenting as severe gingival lesions and swollen gingiva. She had been treated with antibiotics (Zithromax) in the past to control her oral infections and routinely used Chlorhexidine 0.12% rinse solution to control the recolonization of pathogens. Chlorhexidine is used routinely for the control of gingival infection/inflammations as an adjunct to other oral hygiene measures. There are, however side-effects with Chlorhexidine, i.e. staining of teeth, loss of taste and a burning sensation. The alcohol content in the Chlorhexidine solution may also cause some problems for patients. The potential burning sensation was in this case problematic and the patient was not compliant with Chlorhexidine rinsing.

The treatment routine was changed and she was asked to rinse with 0.2% w/v delmopinol once daily for 14 days (instead of chlorhexidine). She had been given a professional cleaning of her teeth prior to rinsing with Delmopinol but no systemic antibiotics were prescribed. Zithromax was used 6 months prior the current treatment.

The following clinical observations were made:
1. During the two weeks no staining or burning sensation as side-effects were reported by the patient who informed the clinicians that she preferred rinsing with Delmopinol.
2. The extent of gingival inflammation was clinically reduced as evidenced by less bleeding on probing and, by visual inspection, less gingival swelling and the absence of ulcerations.

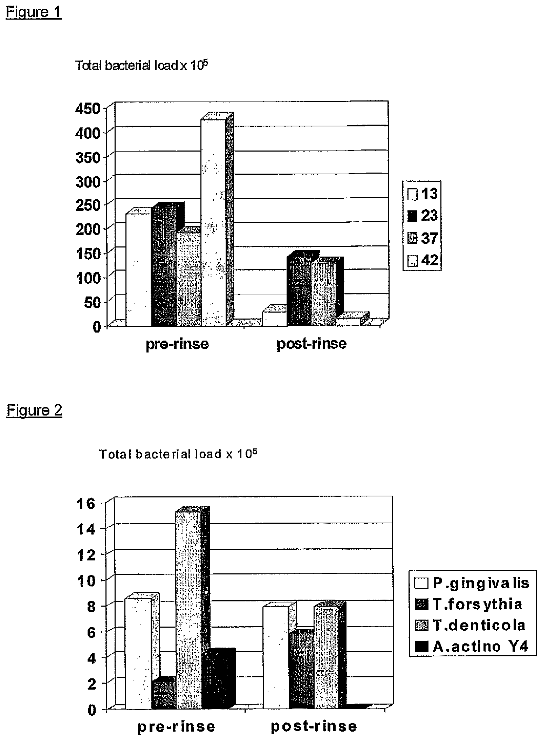

A significant reduction of the total bacterial load from sub-gingival samples taken from four selected sub-gingival pockets (pocket numbers 13, 23, 37 and 42) was seen in samples taken after the 2 week delmopinol period compared to samples taken before the use of delmopinol (see Figure 1).

In addition, a reduction of some key pathogens were found. While the levels of *Porphyromonas gingivalis* and *Tannerella forsythia* were not reduced, the levels of *Treponema denticola* and *Aggregatibaoter actinomycetemcomitans* were significantly reduced (*A.actinomycetemcomitans* was in fact eliminated). See Figure 2.

In summary, it appears that rinsing with delmopinol has an advantage over Chlorhexidine for treating subgingival infection. The lack of staining and the lack of burning improve compliance with the rinse. Secondly, rinsing with delmopinol appears to have an overall positive impact on the subgingival microbiota. Delmopinol is therefore clinically useful in controlling a sub-gingival infection.

## Claims

1. A morpholino compound having the general formula (I) 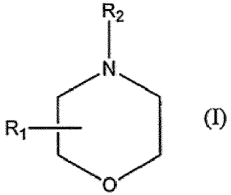 wherein R₁ is a straight or branched alkyl group containing 8 to 16 carbon atoms at the 2- or 3-position of the morpholino ring, and R₂ is a straight or branched alkyl group containing 2 to 10 carbon atoms, substituted with a hydroxy group except in the alpha-position, or a pharmaceutically acceptable salt thereof, for use in treating an infected sub-gingival pocket, wherein the sub-gingival pocket is 4mm deep or more.

2. A morpholino compound for use according to claim 1, wherein the sum of the carbon atoms in the groups R₁ and R₂ of the morpholino compound is at least 10, preferably between 10 and 20.

3. A morpholino compound for use according to claim 1 or 2, wherein R₂ of the morpholino compound terminates with the hydroxy group.

4. A morpholino compound for use according to any preceding claim, wherein the morpholino compound is 3-(4-propyl-heptyl)-4-(2-hydroxyethyl)morpholine.

5. A morpholino compound for use according to any preceding claim, wherein the infected sub-gingival pocket is infected by a bacterial complex defined herein as the red complex.

6. A morpholino compound for use according to any preceding claim, wherein the infected sub-gingival pocket comprises any of *Tannerella forsythensis, Porphyromonas gingivalis, Treponema denticola* or *Actinobacillus actinomycetemcomitans.*

7. A morpholino compound for use according to any preceding claim, wherein the sub-gingival infection is **characterised by** periodontitis.

8. A morpholino compound for use according to any preceding claim, wherein the sub-gingival infection is **characterised by** chronic periodontitis.

9. A morpholino compound for use according to any preceding claim, wherein the compound of formula (I) is for use in combination with an anti-microbial agent.

10. A morpholino compound for use according to any preceding claim, wherein the compound of formula (I) is for use in combination with an anti-inflammatory agent.

11. A morpholino compound for use according to claim 1, wherein the compound of formula (I) is for use in a pet or a farm animal.

12. A morpholino compound for use according to claim 1, wherein the compound of formula (I) is in the form of a mouthwash, toothpaste, gel, dentrifice or gum.

13. A morpholino compound for use according to claim 1, wherein the compound of formula (I) is in a composition comprising one or more non-active ingredients selected from solvents, colourings, flavourings and sweeteners.

14. A morpholino compound for use according to claim 13 wherein said compound of formula (I) is present in the composition in a concentration of between about 0.01 to about 10 % (w/v).

15. A morpholino compound for use according to claim 13 or claim 14, wherein the composition further comprises an anti-microbial agent.

16. A morpholino compound for use according to claim 13 or claim 14, wherein the composition further comprises an anti-inflammatory agent.

17. A morpholino compound for use according to claim 13 or claim 14, wherein the composition further comprises an anti-microbial agent and an anti-inflammatory agent.

## Patentansprüche

1. Morpholinoverbindung mit der allgemeinen Formel (I) 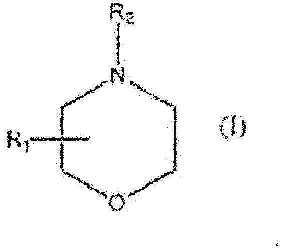 wobei R₁ eine geradkettige oder verzweigte Alkylgruppe mit 8 bis 16 Kohlenstoffatomen in der 2- oder 3-Position des Mopholinorings ist und R₂ eine geradkettige oder verzweigte Alkylgruppe mit 2 bis 10 Kohlenstoffatomen, substituiert durch eine Hydroxygruppe, ausgenommen in der alpha-Position, ist, oder ein pharmazeutisch verträgliches Salz davon zur Verwendung bei der Behandlung einer infizierten subgingivalen Tasche, wobei die subgingivale Tasche 4 mm tief oder tiefer ist.

2. Morpholinoverbindung zur Verwendung nach Anspruch 1, wobei die Summe der Kohlenstoffatome in den Gruppen R₁ und R₂ der Morpholinoverbindung mindestens 10, vorzugsweise zwischen 10 und 20 beträgt.

3. Morpholinoverbindung zur Verwendung nach Anspruch 1 oder 2, wobei R₂ der Morpholinoverbindung mit der Hydroxygruppe endet.

4. Morpholinoverbindung zur Verwendung nach einem der vorangehenden Ansprüche, wobei die Morpholinoverbindung 3-(4-Propylheptyl)-4-(2-Hydroxyethyl)morpholin ist.

5. Morpholinoverbindung zur Verwendung nach einem der vorangehenden Ansprüche, wobei die infizierte subgingivale Tasche mit einem Bakterienkomplex infiziert ist, der hier als der rote Komplex definiert ist.

6. Morpholinoverbindung zur Verwendung nach einem der vorangehenden Ansprüche, wobei die infizierte subgingivale Tasche eines von *Tannerella forsythensis, Porphyromonas gingivalis, Treponema denticola* oder *Actinobacillus actinomycetemcomitans* umfasst.

7. Morpholinoverbindung zur Verwendung nach einem der vorangehenden Ansprüche, wobei die subgingivale Infektion durch Parodontitis charakterisiert ist.

8. Morpholinoverbindung zur Verwendung nach einem der vorangehenden Ansprüche, wobei die subgingivale Infektion durch chronische Parodontitis charakterisiert ist.

9. Morpholinoverbindung zur Verwendung nach einem der vorangehenden Ansprüche, wobei die Verbindung mit der Formel (I) zur Verwendung in Kombination mit einem antimikrobiellen Agens ist.

10. Morpholinoverbindung zur Verwendung nach einem der vorangehenden Ansprüche, wobei die Verbindung mit der Formel (I) zur Verwendung in Kombination mit einem entzündungshemmenden Agens ist.

11. Morpholinoverbindung zur Verwendung nach Anspruch 1, wobei die Verbindung mit der Formel (I) zur Verwendung in einem Haustier oder Nutztier ist.

12. Morpholinoverbindung zur Verwendung nach Anspruch 1, wobei die Verbindung mit der Formel (I) in Form eines Mundwassers, einer Zahnpasta, eines Gels, eines Zahnputzmittels oder eines Gummis vorliegt.

13. Morpholinoverbindung zur Verwendung nach Anspruch 1, wobei die Verbindung mit der Formel (I) in einer Zusammensetzung ist, die ein oder mehrere nicht-aktive Inhaltsstoffe umfasst, die ausgewählt sind aus Lösungsmitteln, Farbstoffen, Geschmacksstoffen und Süßungsmitteln.

14. Morpholinoverbindung zur Verwendung nach Anspruch 13, wobei die Verbindung mit der Formel (I) in der Zusammensetzung in einer Konzentration von zwischen etwa 0,01 bis etwa 10% (w/v) vorliegt.

15. Morpholinoverbindung zur Verwendung nach Anspruch 13 oder Anspruch 14, wobei die Zusammensetzung des Weiteren ein antimikrobielles Agens umfasst.

16. Morpholinoverbindung zur Verwendung nach Anspruch 13 oder Anspruch 14, wobei die Zusammensetzung des Weiteren ein entzündungshemmendes Agens umfasst.

17. Morpholinoverbindung zur Verwendung nach Anspruch 13 oder Anspruch 14, wobei die Zusammensetzung des Weiteren ein antimikrobielles Agens und ein entzündungshemmendes Agens umfasst.

## Revendications

1. Composé morpholino ayant la formule générale (I) 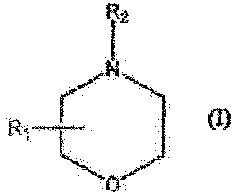 où R₁ est un groupe alkyle linéaire ou ramifié contenant 8 à 16 atomes de carbone à la position 2 ou 3 du cycle morpholino, et R₂ est un groupe alkyle linéaire ou ramifié contenant 2 à 10 atomes de carbone, substitué avec un groupe hydroxy sauf dans la position alpha, ou sel pharmaceutiquement acceptable de celui-ci, destiné à être utilisé dans le traitement d'une poche sous-gingivale infectée, où la poche sous-gingivale est profonde de 4 mm ou plus.

2. Composé morpholino destiné à être utilisé selon la revendication 1, où la somme des atomes de carbone dans les groupes R₁ et R₂ du composé morpholino est au moins 10, de préférence entre 10 et 20.

3. Composé morpholino destiné à être utilisé selon la revendication 1 ou 2, où R₂ du composé morpholino se termine avec le groupe hydroxy.

4. Composé morpholino destiné à être utilisé selon l'une quelconque des revendications précédentes, où le composé morpholino est la 3-(4-propyl-heptyl)-4-(2-hydroxyéthyl)morpholine.

5. Composé morpholino destiné à être utilisé selon l'une quelconque des revendications précédentes, où la poche sous-gingivale infectée est infectée par un complexe bactérien défini ici comme étant le complexe rouge.

6. Composé morpholino destiné à être utilisé selon l'une quelconque des revendications précédentes, où la poche sous-gingivale infectée comprend l'un quelconque de *Tannerella forsythensis*, *Porphyromonas gingivalis*, *Treponema denticola* et *Actinobacillus actinomycetemcomitans.*

7. Composé morpholino destiné à être utilisé selon l'une quelconque des revendications précédentes, où l'infection sous-gingivale est **caractérisée par** une périodontite.

8. Composé morpholino destiné à être utilisé selon l'une quelconque des revendications précédentes, où l'infection sous-gingivale est **caractérisée par** une périodontite chronique.

9. Composé morpholino destiné à être utilisé selon l'une quelconque des revendications précédentes, où le composé de formule (I) est destiné à être utilisé en combinaison avec un agent antimicrobien.

10. Composé morpholino destiné à être utilisé selon l'une quelconque des revendications précédentes, où le composé de formule (I) est destiné à être utilisé en combinaison avec un agent anti-inflammatoire.

11. Composé morpholino destiné à être utilisé selon la revendication 1, où le composé de formule (I) est destiné à être utilisé chez un animal familier ou un animal de ferme.

12. Composé morpholino destiné à être utilisé selon la revendication 1, où le composé de formule (I) est sous forme d'un bain de bouche, d'une pâte dentifrice, d'un gel, d'un dentifrice ou d'une gomme.

13. Composé morpholino destiné à être utilisé selon la revendication 1, où le composé de formule (I) est dans une composition comprenant un ou plusieurs ingrédients non actifs choisis parmi les solvants, les colorants, les aromatisants et les édulcorants.

14. Composé morpholino destiné à être utilisé selon la revendication 13 où ledit composé de formule (I) est présent dans la composition en une concentration entre environ 0,01 et environ 10 % (p/v).

15. Composé morpholino destiné à être utilisé selon la revendication 13 ou la revendication 14, où la composition comprend en outre un agent antimicrobien.

16. Composé morpholino destiné à être utilisé selon la revendication 13 ou la revendication 14, où la composition comprend en outre un agent anti-inflammatoire.

17. Composé morpholino destiné à être utilisé selon la revendication 13 ou la revendication 14, où la composition comprend en outre un agent antimicrobien et un agent anti-inflammatoire.
